# EUROPEAN PATENT SPECIFICATION

(11) **EP 3 064 773 B1**
(45) Date of publication and mention of the grant of the patent: **12.06.2019**
(21) Application number: 16163481.1
(22) Date of filing: 01.04.2013
(51) Int. Cl.: F04B 41/06, A61M 16/00, F04B 45/04, F04B 49/10, A61M 16/04, A61M 16/06, A61M 16/20, F04B 9/10, A61M 16/12

(54) **PUMP UNIT AND RESPIRATORY ASSISTANCE DEVICE**
PUMPENEINHEIT UND ATEMHILFEVORRICHTUNG
UNITÉ DE POMPE ET DISPOSITIF D'ASSISTANCE RESPIRATOIRE

(30) Priority: 02.04.2012 JP 2012083615
(43) Date of publication of application: 07.09.2016
(62) Divisional of application: 13771805.2
(73) Proprietor: Metran Co., Ltd., Kawaguchi-shi, Saitama 332-0015 (JP)
(72) Inventor: NITTA, Kazufuku, Kawaguchi-shi,, Saitama 332-0015 (JP)
(74) Representative: TBK

(56) References cited:
- EP-A1- 2 529 780
- US-A- 6 106 245
- US-A1- 2004 202 554
- US-A1- 2011 125 086
- KAN J ET AL: "Development of serial-connection piezoelectric pumps", SENSORS AND ACTUATORS A, ELSEVIER SEQUOIA S.A., LAUSANNE, CH, vol. 144, no. 2, 15 June 2008 (2008-06-15) , pages 321-327, XP022664310, ISSN: 0924-4247, DOI: 10.1016/J.SNA.2008.01.016 [retrieved on 2008-02-06]

## Description

The present invention relates to a pump unit for transporting a fluid by means of a micro pump and a respiratory assistance device employing the same.

### Background Art

In medical practice, respiratory assistance devices such as artificial respirators are employed. Types of such a respiratory assistance device employ: a controlled ventilation (Controlled Ventilation) method employed for a patient in the absence of spontaneous breathing (a patient under general anesthesia, during cardiopulmonary resuscitation, or in a critical condition); an assisted ventilation (Assisted Ventilation) method in which a positive pressure is created in an air passage in synchronization with the spontaneous breathing of a patient; a partial assisted (Assist/Control) method employing the assisted ventilation and the controlled ventilation in combination; a high frequency oscillation ventilation (high frequency oscillation) with which a very small amount of a single ventilation, 1 to 2 ml/kg, can be achieved by causing a gas supplied by an air passage to oscillate at a frequency of 5 to 40 Hz, etc.

Such a respiratory assistance device is employed also for a patient suffering from a respiratory disorder during sleep. This respiratory disorder is caused by the blockage of an air passage as a result of relaxation of the muscle of the air passage during sleep and the resultant retraction of the posterior part of a tongue or a soft palate. Applying a positive pressure to the air passage of the patient suffering from this type of respiratory disorder can alleviate its symptoms.

Any of these respiratory assistance devices requires a pump unit for creating a positive pressure in an air passage. A blower for transporting a gas by rotating a fan, a cylinder pump for transporting a gas by causing a piston to reciprocate, or the like is employed as a power source for this pump unit.

### Summary of Invention

In the conventional respiratory assistance device, however, the pump unit is housed in a box-shaped housing and is placed beside a user when used due to a relatively large size thereof. Thus, there is a problem in that the downsizing of the respiratory assistance device is difficult to achieve.

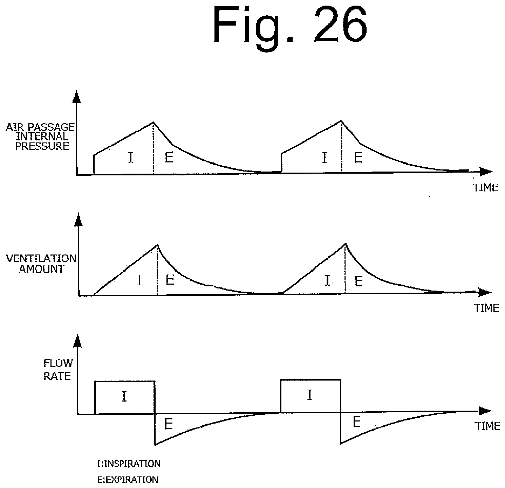

Moreover, according to the pump unit employed in the respiratory assistance device, during an inspiratory operation, a pressure is initially increased (a positive pressure is created) rapidly at a high flow rate and then a constant flow rate is maintained while assisting inspiration by further increasing the pressure as shown in Fig. 26, for example. During an expiratory operation, a pressure is decreased (a negative pressure is created) rapidly at a high flow rate. Once the pressure is lowered, the flow rate is controlled so as to be gradually decreased in order to avoid a burden on a lung. Such control is merely an example and various control modes are required in practice. In order to perform fine control of this type, however, a relatively large blower or cylinder pump needs to be employed and its pressure and its flow rate should be capable of being changed as desired. Thus, there is a problem in that the downsizing of the pump unit is further complicated.

Document US 2004/0202554 A1 discloses a micro pump being configured by a cell having only one port. By changing the cell's volume, a system fluid present in the cell is made to protrude out of the cell in order to displace a fluid in a flow channel. The fluid in the flow channel is insoluble in the system fluid. By again changing the cell's volume, the protruding system fluid is again retracted into the cell, enabling suction of additional fluid into the flow channel.

Document US 6,106,245 A discloses mesopump sets having plural cells. The mesopump sets can be connected in a parallel or in a serial manner (see col. 4, lines 10-14).

Post published document EP 2 529 780 A1 discloses a pump unit breathing assistant device. Micro pumps are arranged in a lattice pattern with rows and columns, and a discharge port of at least a micro pump arranged in a most downstream row is directly connected to an integrated discharge port.

The present invention has been made in view of the aforementioned problems and it is the object of the present invention to provide a pump unit capable of achieving significant downsizing while maintaining an ability to control its pressure and its flow rate as desired and a respiratory assistance device employing the same.

The object of the invention is achieved by a pump unit according to claim 1.

As a result of intensive studies made by the present inventor, the aforementioned object is achieved by the following means.

More specifically, a pump unit achieving the aforementioned object comprising: a body provided with an inlet and an outlet for a fluid; and a pump group composed of a plurality of micro pumps arranged in the body on a plane so that discharge ports and suction ports of the micro pumps are aligned in a same direction, for allowing a fluid entering through the inlet to exit from the outlet by discharging the fluid from the suction ports to the discharge ports in one direction, wherein the body includes an inlet package having the inlet and an outlet package having the outlet to hold the plurality of arranged micro pumps between them, the inlet package collectively holds the plurality of micro pumps from a side of the suction ports and has a structure that allows the suction ports of the micro pumps and the inlet of the body to be communicated with or closed off from each other, and the outlet package collectively holds the plurality of micro pumps from a side of the discharge ports and has a structure that allows the discharge ports of the micro pumps and the outlet of the body to be communicated with or closed off from each other.

The body is provided with a depressed portion for housing the micro pump.

The micro pump includes a power-feeding terminal for feeding power to a pump device contained therein, and the depressed portion is provided with a line electrically connecting to the power-feeding terminal of the micro pump housed in the depressed portion.

Preferably, a pump unit achieving the aforementioned object includes: a body provided with an inlet and an outlet for a fluid; and a pump group composed of a plurality of micro pumps arranged in the body, for allowing a fluid entering through the inlet to exit from the outlet. The pump group includes: a micro pump positioned in most upstream in a serial state; a micro pump positioned in most downstream in the serial state; and a micro pump positioned in middle in the serial state. The body includes: an inlet direct-connecting flow passage directly connecting a suction port of the micro pump positioned in the most upstream with the inlet; an outlet direct-connecting flow passage directly connecting a discharge port of the micro pump positioned in the most downstream with the outlet; and a flow passage forming mechanism that connects the micro pumps constituting the pump group. The flow passage forming mechanism is switchable between the serial state in which the micro pump positioned in the most upstream, the micro pump positioned in the middle, and the micro pump positioned in the most downstream are connected in this order and a parallel state in which a branched passage connecting between a suction port of the micro pump positioned in the middle or in the most downstream and the inlet is formed and a confluent passage connecting between a discharge port of the micro pump positioned in the most upstream or in the middle and the outlet is formed.

Preferably, a flow passage forming control part for controlling the flow passage forming mechanism is provided. Moreover, the flow passage forming mechanism preferably includes: first flow passage forming means that allows the suction ports of the micro pumps positioned in the middle and in the most downstream and the inlet of the body to be communicated with or closed off from each other; second flow passage forming means that allows the discharge port of the micro pump on an upstream side and the suction port of the micro pump on a downstream side to be communicated with or closed off from each other in the micro pumps connected in the order of the most upstream, the middle, and the most downstream; and third flow passage forming means that allows the discharge ports of the micro pumps positioned in the most upstream and in the middle and the outlet of the body to be communicated with or closed off from each other.

The micro pumps constituting the pump group may be arranged so as to be stacked one another or may be arranged in a lattice pattern. Moreover, a row bypass flow passage that connects suction ports of a plurality of the micro pumps arranged in a row direction and connects discharge ports of the plurality of the micro pumps arranged in the row direction and a row bypass flow passage opening and closing device for opening and closing the row bypass flow passage are preferably provided. Furthermore, a column bypass flow passage that connects suction ports of a plurality of the micro pumps arranged in a column direction and connects discharge ports of the plurality of the micro pumps arranged in the column direction and a column bypass flow passage opening and closing device for opening and closing the column bypass flow passage are preferably provided.

The flow passage forming control part preferably includes: a failure detecting part for detecting a failure of the micro pump; a pump substitution control part for determining whether or not there is a micro pump which can be substituted for a broken micro pump; and a bypass control part for controlling, when it is determined that there is the substitution micro pump, the row bypass flow passage opening and closing device or the column bypass flow passage opening and closing device so that the fluid flowing toward the micro pump specified by a failure signal is sent to the substitution micro pump and the fluid exiting from the substitution micro pump is sent to the micro pump subsequent to the micro pump specified by the failure signal.

Preferably, a warning device capable of issuing a warning is provided and the flow passage forming control part includes a warning notification part for giving a warning by means of the warning device when it is determined that the substitution micro pump does not exist.

Preferably, the body is provided with a depressed portion for housing the micro pump. Moreover, the micro pump preferably includes a power-feeding terminal for feeding power to a pump device contained therein, and the depressed portion is preferably provided with a line electrically connecting to the power-feeding terminal of the micro pump housed in the depressed portion.

Preferably, the body includes an inlet package having the inlet and an outlet package having the outlet, the first flow passage forming means is provided in the inlet package, and the third flow passage forming means is provided in the outlet package.

A respiratory assistance device achieving the aforementioned object includes: a flow passage through which an expiratory or inspiratory gas passes; a nozzle disposed in the flow passage, for jetting an acceleration gas in an expiratory or inspiratory direction; and the above-described pump unit fixed around the flow passage, for supplying the acceleration gas to the nozzle.

The present invention achieves an excellent effect such that the pump unit can be significantly downsized while maintaining an ability to control a pressure and a flow rate as desired.

### Brief Description of Drawings

Fig.1 is a perspective view illustrating an outline of a pump unit.
Fig. 2 is a perspective view illustrating the outline of the pump unit.
Fig. 3 is an exploded perspective view illustrating the outline of the pump unit.
Fig. 4 is a perspective view illustrating an outline of a micro pump.
Fig. 5 is a cross-sectional view illustrating the outline of the micro pump.
Fig. 6 is a graph showing pressure-flow rate lines for the micro pump.
Fig. 7 is a plan view illustrating an outline of micro pumps arranged in a lattice pattern on an upper surface of an inlet-side housing plate.
Fig. 8 is a connection diagram of micro pumps contained in the pump unit.
Fig. 9 is a cross-sectional view of the pump unit.
Fig. 10 is a configuration diagram illustrating an outline of a controller.
Fig. 11 is a functional block diagram illustrating the outline of the controller.
Fig. 12 is a connection diagram illustrating an outline of a pump unit in a pressure preferential transporting state.
Fig. 13 is a connection diagram illustrating an outline of the pump unit in a flow rate preferential transporting state.
Fig. 14 is a connection diagram illustrating an outline of a pump unit including spare micro pumps.
Fig. 15 is a connection diagram illustrating an outline of a pump unit in a state where a flow passage has been switched so that a fluid is allowed to flow to a spare micro pump instead of a micro pump in a failure state.
Fig. 16 is a connection diagram illustrating an outline of a pump unit in a state where a flow passage has been switched so that a fluid is allowed to flow to a spare micro pump instead of a micro pump in a failure state.
Fig. 17 is a perspective view illustrating an outline of a pump unit.
Fig. 18 is a cross-sectional view illustrating the outline of the pump unit.
Fig. 19 is a cross-sectional view illustrating the outline of the pump unit.
Fig. 20 is a perspective view illustrating an outline of a plurality of micro pumps housed in a housing of the pump unit and a flow passage block disposed between the plurality of micro pumps.
Fig. 21 is a perspective view illustrating an outline of a pump unit.
Fig. 22A is a cross-sectional view illustrating an outline of a respiratory assistance device.
Fig. 22B is a cross-sectional view as viewed along arrows B-B in Fig. 22A.
Fig. 23A is a cross-sectional view illustrating a control example of the respiratory assistance device.
Fig. 23B is a cross-sectional view illustrating a control example of the respiratory assistance device.
Fig. 24 is a cross-sectional view illustrating an outline of another respiratory assistance device.
Fig. 25 is a cross-sectional view illustrating an outline of another respiratory assistance device.
Fig. 26 shows graphs illustrating a control example of a pressure and a flow rate in a typical respiratory assistance device.

Embodiments of the present invention will now be described below with reference to the accompanying drawings.

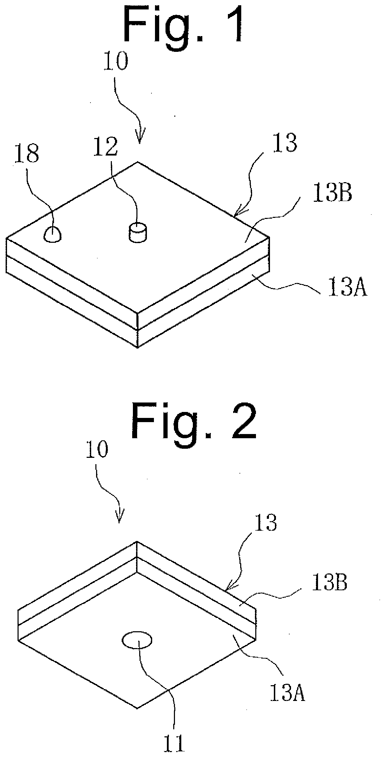

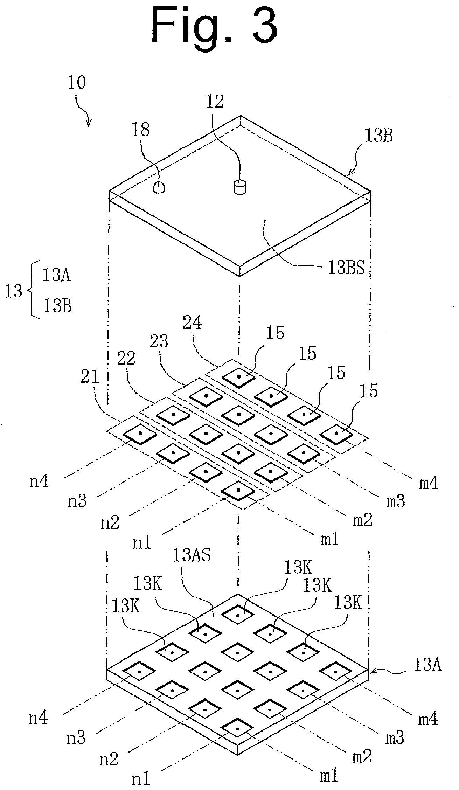

As shown in Figs. 1 to 2, a pump unit 10 includes: a plate-like housing 13 having an inlet 11 and an outlet 12; micro pumps 15 (see Fig. 3) housed in the housing 13; and a light-emitting diode 18. The pump unit 10 sucks a fluid in from the inlet 11 and lets the sucked fluid out from the outlet 12 by means of the micro pumps 15 (see Fig. 3).

As shown in Fig. 3, the housing 13 has an inlet-side housing plate 13A and an outlet-side housing plate 13B. Depressed portions 13K to which the micro pumps 15 are attached are formed on a surface 13AS of the inlet-side housing plate 13A. On the surface 13AS, the depressed portions 13K are arranged in a lattice pattern. Although the diagrammatic illustration thereof is omitted, depressed portions to which the micro pumps 15 are attached are formed also on a surface 13BS of the outlet-side housing plate 13B. The depressed portions on the surface 13BS are provided at positions directly facing the depressed portions 13K when the housing plates 13A and 13B are overlapped with each other with the surfaces 13AS and 13BS facing each other. When the housing plates 13A and 13B are overlapped with each other with the surfaces 13AS and 13BS facing each other, housing spaces for the micro pumps 15 are formed by the depressed portions 13K on the inlet-side housing plate 13A and the depressed portions on the outlet-side housing plate 13B. Therefore, by placing the micro pumps 15 on the depressed portions provided on any one of the housing plates 13A and 13B, the micro pumps 15 are arranged in a lattice pattern of m rows × n columns (4 rows × 4 columns, for example). Thereafter, by overlapping the housing plates 13A and 13B with each other with the surfaces 13AS and 13BS facing each other, the micro pumps 15 are contained in the housing 13 while keeping the lattice arrangement.

The plurality of micro pumps 15 (pump group) contained in the housing 13 form: a most upstream row group 21 composed of the micro pumps 15 arranged in the most upstream row (the row m1 in the figure); a most downstream row group 24 composed of the micro pumps 15 arranged in the most downstream row (the row m4 in the figure); and middle row groups 22 and 23 each composed of the micro pumps arranged in the row direction (the row m2 and the row m3 in the figure) between the most upstream row group 21 and the most downstream row group 24.

A flow passage for a fluid is formed in the housing 13. The flow passage is formed so as to connect between suction ports and discharge ports of the micro pumps 15 contained in the housing 13 and so that the fluid is transported in the housing 13 from the inlet 11 to the outlet 12. The flow passage will be described later.

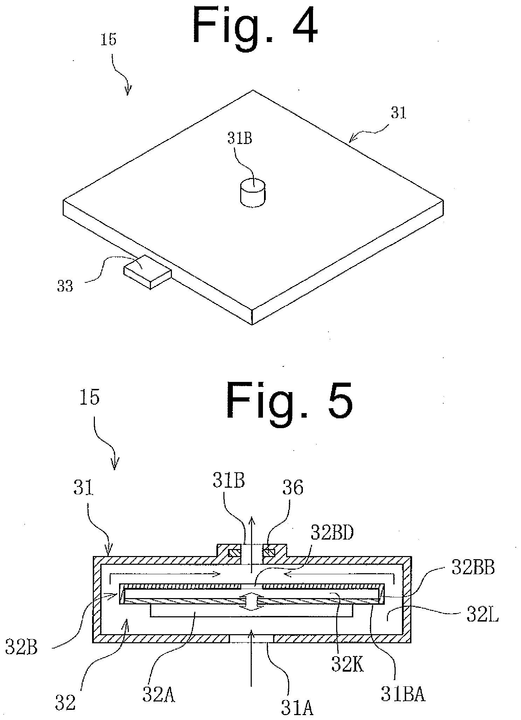

A micro pump proposed in Patent Literature WO 2008/069266, for example, can be employed as the micro pump 15. As shown in Figs. 4 and 5, the micro pump 15 includes: a case 31 having a suction port 31A and a discharge port 31B; a pump device 32 contained in the case 31, for transporting a gas from the suction port 31A to the discharge port 31B; and a power-feeding terminal 33 exposed to the outside of the case 31.

As shown in Fig. 5, the pump device 32 is electrically connected to the power-feeding terminal 33. The pump device 32 includes: a piezoelectric element 32A deformable when a voltage is applied; and a deformable box 32B deformable by the actuation of the piezoelectric element. The deformable box 32B includes a diaphragm 32BA and an oscillation wall 32BB. The diaphragm 32BA is provided in a portion of the deformable box 32B facing the suction port 31A. The oscillation wall 32BB is provided in a portion of the deformable box 32B facing the discharge port 31B. A primary blower chamber 32K is formed between the diaphragm 32BA and the oscillation wall 32BB. The piezoelectric element 32A is attached to a surface of the diaphragm 32BA facing the suction port 31A. Furthermore, in the oscillation wall 32BB, an opening 32BD through which the fluid is moved between the inside and outside of the primary blower chamber 32K is formed at a position directly facing the discharge port 31B.

When the diaphragm 32BA is oscillated by the piezoelectric element 32A, the fluid is moved between a secondary blower chamber 32L formed by the case 31 and the pump device 32 and the primary blower chamber 32K. Such a fluid movement causes the oscillation wall 32BB to resonate. The oscillation of the diaphragm 32BA and the oscillation wall 32BB causes the fluid to be sucked in from the suction port 31A. The fluid sucked in from the suction port 31A is passed through the secondary blower chamber 32L and emitted from the discharge port 31B. The micro pump 15 is suitable for use as a blower for transporting a fluid. The micro pump 15 can transport a fluid without the use of a check valve.

A frequency of the diaphragm 32BA is greater than or equal to 1 kHz, for example, and preferably in a range between 18 kHz and 27 kHz. Moreover, the frequency of the diaphragm 32BA is preferably in an inaudible range. Consequently, when a patient is equipped with a device including the pump device 32 (for example, a respiratory assistance device), the patient cannot hear the operation noise of the pump device 32. Thus, this keeps the patient free from suffering discomfort caused by the operation noise.

The micro pump 15 further includes a sensor unit 36 for detecting a failure of the pump device 32. The sensor unit 36 includes: a pressure sensor for detecting a static pressure P of a fluid at the discharge port 31B; and a flow sensor for detecting a flow rate Q of the fluid at the discharge port 31B.

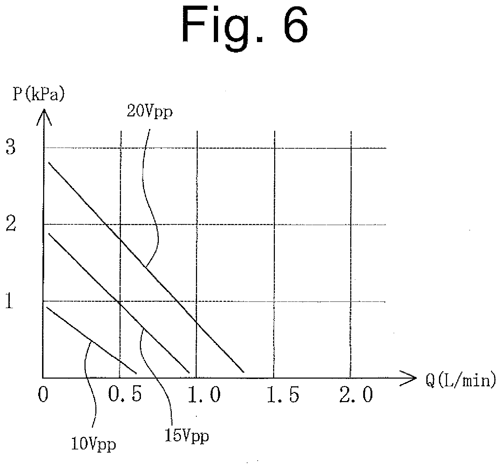

The micro pump 15 is formed in a plate shape and extremely small (about 20 mm in length × 20 mm in width × 2 mm in thickness, for example). The micro pump 15 can still transport a fluid of about 1 L/min at maximum when the input sine wave is set at 26 kHz under 15 Vpp (Volt peak to peak) and can obtain a static pressure of 2 kPa at maximum (see Fig. 6) .

The micro pump 15 transports a fluid by means of the oscillation of the diaphragm 32BA caused by the piezoelectric element 32A. Thus, there is naturally a limit in the volume of a fluid the micro pump 15 can transport. The static pressure-vs-flow rate characteristics thereof also show a trend as shown in Fig. 6 (for example, a linear function with a negative proportionality multiplier or something similar). In order to obtain a static pressure of about 1 kPa, for example, the required flow rate Q is 0.5 L/min. Setting the input sine wave at 10 Vpp or 20 Vpp causes the amplitude of the piezoelectric element 32A to change. Thus, the flow rate Q and the static pressure P according to the input sine wave can be obtained. More specifically, if the Vpp of the input sine wave is smoothly changed, the flow rate Q and the static pressure P can be smoothly changed. Alternatively, if the frequency of the input sine wave is changed, the flow rate Q and the static pressure P can be changed. More specifically, if the frequency of the input sine wave is smoothly changed, the flow rate Q and the static pressure P can be smoothly changed. Note, however, that the flow rate Q and the static pressure P each have an upper limit according to the capacity of the piezoelectric element 32A and the strength or durability of components of the micro pump 15. The micro pump 15 is normally used at a rated Vpp and a rated frequency.

Note that the micro pump 15 may have a monomorph (unimorph) structure as described above in which the single piezoelectric element 32A is attached to the diaphragm 32BA or a bimorph structure in which two piezoelectric elements 32A are attached together in order to increase the amount of oscillation. An appropriate structure of the micro pump 15 can be adopted in accordance with its purpose such as the transportation of a fluid. While the micro pump 15 can transport a gas without employing a check valve, the micro pump 15 may be replaced by a micro pump including a check valve at the suction port or the discharge port.

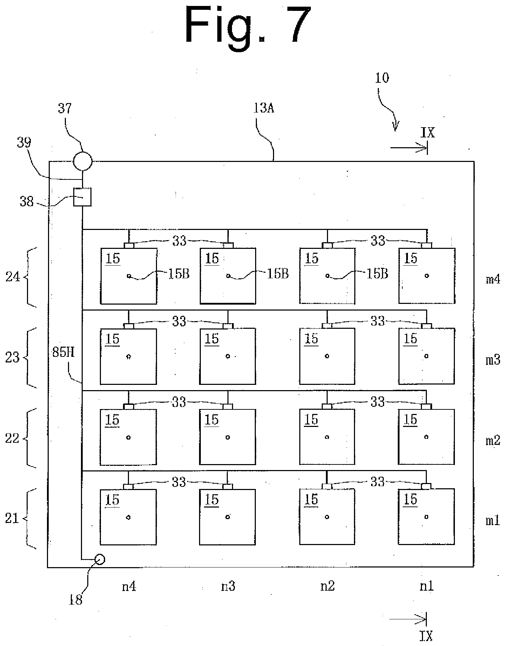

As shown in Figs. 3 and 7, the housing 13 includes: an external power-supply terminal 37; a controller 38; and a line 39. The external power-supply terminal 37 is provided so as to be exposed on the housing 13. The controller 38 and the line 39 are provided in the inlet-side housing plate 13A. The line 39 electrically connects between the external power-supply terminal 37 and the controller 38. A bus 85H electrically connects the controller 38, the light-emitting diode 18, and the power-feeding terminals 33 provided in the respective micro pumps 15. The detail of the controller 38 will be described later.

The housing 13 has an inlet direct-connecting mechanism, an outlet direct-connecting mechanism, and a flow passage forming mechanism connecting between the inlet direct-connecting mechanism and the outlet direct-connecting mechanism.

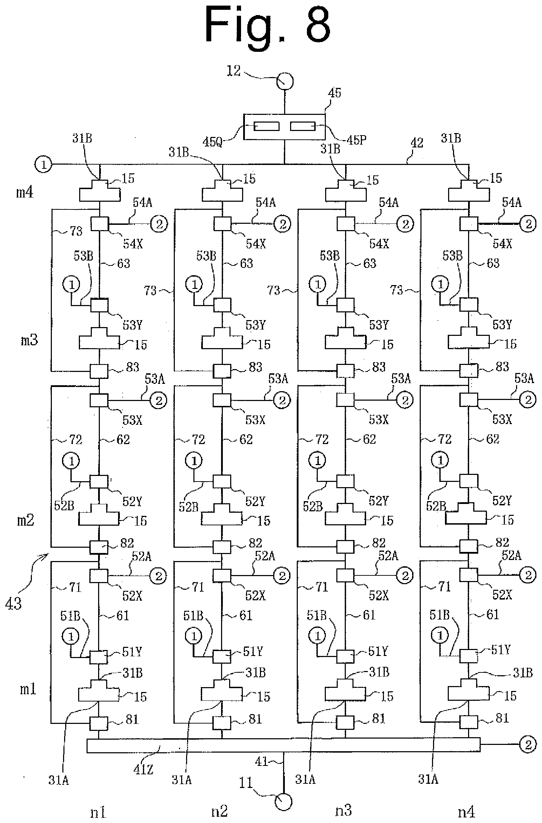

As shown in Figs. 8 to 9, the inlet direct-connecting mechanism is an inlet direct-connecting flow passage 41 directly connecting the suction ports 31A of all the micro pumps 15 that belong to the most upstream row group 21 (the row m1 in the figure) with the inlet 11. The inlet direct-connecting flow passage 41 is formed in the inlet-side housing plate 13A. The inlet direct-connecting flow passage 41 is provided with a switching valve 41Z. The switching valve 41Z is switchable between a parallel state in which the suction ports 31A of a plurality of micro pumps 15 that belong to the most upstream row group 21 (the row m1 in the figure) are communicated with the inlet 11 and a serial state in which the suction port 31A of any one of the micro pumps 15 that belong to the most upstream row group 21 (the row m1 in the figure) is communicated with the inlet 11. Note that when the switching valve 41Z is in the parallel state, the suction ports 31A of all the micro pumps 15 that belong to the most upstream row group 21 (the row m1 in the figure) may be communicated with the inlet 11 or the suction ports 31A for a part of the micro pumps 15 that belong to the most upstream row group 21 (the row m1 in the figure) may be communicated with the inlet 11 while the suction ports 31A for the remaining micro pumps 15 may not be communicated with the inlet 11.

The outlet direct-connecting mechanism is an outlet direct-connecting flow passage 42 directly connecting the discharge ports 31B in the most downstream row group 24 (the row m4 in the figure) with the outlet 12. The outlet direct-connecting flow passage 42 is formed in the outlet-side housing plate 13B.

Moreover, the flow passage forming mechanism is formed in the inlet-side housing plate 13A and the outlet-side housing plate 13B. The flow passage forming mechanism includes: the aforementioned switching valve 41Z; a middle flow passage 43; and an opening and closing mechanism provided in the middle flow passage 43. The middle flow passage 43 includes: a most upstream discharge port flow passage 51B; a middle suction port flow passage 52A; a middle discharge port flow passage 52B; a middle suction port flow passage 53A; a middle discharge port flow passage 53B; a most downstream suction port flow passage 54A; serial flow passages 61 to 63; and column bypass flow passages 71 to 73.

The most upstream discharge port flow passage 51B connects the discharge ports 31B of all the micro pumps 15 that belong to the most upstream row group 21 (the row m1 in the figure) with one another. The middle suction port flow passage 52A connects the suction ports 31A of all the micro pumps 15 that belong to the middle row group 22 (the row m2 in the figure) with one another. The middle discharge port flow passage 52B connects the discharge ports 31B of all the micro pumps 15 that belong to the middle row group 22 (the row m2 in the figure) with one another. Similarly, the middle suction port flow passage 53A connects the suction ports 31A of all the micro pumps 15 that belong to the middle row group 23 (the row m3 in the figure) with one another. The middle discharge port flow passage 53B connects the discharge ports 31B of all the micro pumps 15 that belong to the middle row group 23 (the row m3 in the figure) with one another. The most downstream suction port flow passage 54A connects the suction ports 31A of all the micro pumps 15 that belong to the most downstream row group 24 (the row m4 in the figure) with one another.

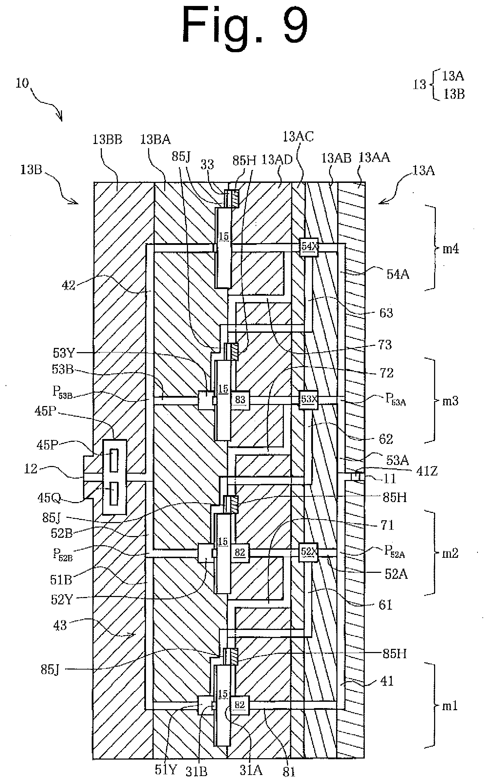

Moreover, the suction port flow passages 52A to 54A are connected to the inlet 11 via the switching valve 41Z and the inlet direct-connecting flow passage 41. The discharge port flow passages 51B to 53B are connected to the outlet 12 via the outlet direct-connecting flow passage 42. Note that the suction port flow passages 52A to 54A may be communicated with the inlet 11 regardless of the state of the switching valve 41Z or may be communicated with the inlet 11 when the switching valve 41Z is in the parallel state and may be closed off from the inlet 11 when the switching valve 41Z is in the serial state. For example, the suction port flow passage 52A and the suction port flow passage 53A are connected to the inlet direct-connecting flow passage 41 at a position P_{52A} (see Fig. 9) and at a position P_{53A} (see Fig. 9), respectively. The suction port flow passage 54A is connected to the flow passage 53A at the position P_{53A}. Similarly, the discharge port flow passage 53B and the discharge port flow passage 52B are communicated with the outlet direct-connecting flow passage 42 at a position P_{53B} (see Fig. 9) and at a position P_{52B} (see Fig. 9), respectively. The discharge port flow passage 51B is communicated with the flow passage 52B at the position P_{52B} (see Fig. 9).

The serial flow passage 61 connects between the discharge port flow passage 51B and the suction port flow passage 52A. Similarly, the serial flow passage 62 connects between the discharge port flow passage 52B and the suction port flow passage 53A. The serial flow passage 63 connects between the discharge port flow passage 53B and the suction port flow passage 54A.

A valve 51Y is provided at a connecting position between the discharge port flow passage 51B and the serial flow passage 61. The valve 51Y can be transitioned between a parallel state in which the serial flow passage 61 is closed while opening the discharge port flow passage 51B positioned downstream (the outlet 12 side) of the valve 51Y and a serial state in which the serial flow passage 61 is opened while closing the discharge port flow passage 51B positioned downstream (the outlet 12 side) of the valve 51Y. Note that the discharge port flow passage 51B positioned upstream (the discharge port 31B side) of the valve 51Y is kept opened in either of the parallel state and the serial state.

Similarly, a valve 52Y is provided at a connecting position between the discharge port flow passage 52B and the flow passage 62 and a valve 53Y is provided at a connecting position between the discharge port flow passage 53B and the serial flow passage 63. The valve 52Y can be transitioned between a parallel state in which the serial flow passage 62 is closed while opening the discharge port flow passage 52B positioned downstream (the outlet 12 side) of the valve 52Y and a serial state in which the serial flow passage 62 is opened while closing the discharge port flow passage 52B positioned downstream (the outlet 12 side) of the valve 52Y. Note that the discharge port flow passage 52B positioned upstream (the discharge port 31B side) of the valve 52Y is kept opened in either of the parallel state and the serial state. Similarly, the valve 53Y can be transitioned between a parallel state in which the serial flow passage 63 is closed while opening the discharge port flow passage 53B positioned downstream (the outlet 12 side) of the valve 53Y and a serial state in which the serial flow passage 63 is opened while closing the discharge port flow passage 53B positioned downstream (the outlet 12 side) of the valve 53Y. Note that the discharge port flow passage 53B positioned upstream (the discharge port 31B side) of the valve 53Y is kept opened in either of the parallel state and the serial state.

A valve 52X is provided at a connecting position between the suction port flow passage 52A and the serial flow passage 61. The valve 52X can be transitioned among a parallel state in which the serial flow passage 61 is closed while the other flow passages are opened, a serial state in which the suction port flow passage 52A positioned upstream (the inlet 11 side) of the valve 52X is closed while the other flow passages are opened, and a bypass state in which the suction port flow passage 52A positioned downstream of the valve 52X is closed while the other flow passages are opened. Similarly, a valve 53X is provided at a connecting position between the suction port flow passage 53A and the serial flow passage 62 and a valve 54X is provided at a connecting position between the suction port flow passage 54A and the serial flow passage 63. The valve 53X can be transitioned among a parallel state in which the serial flow passage 62 is closed while the other flow passages are opened, a serial state in which the suction port flow passage 53A positioned upstream (the inlet 11 side) of the valve 53X is closed while the other flow passages are opened, and a bypass state in which the suction port flow passage 53A positioned downstream of the valve 53X is closed while the other flow passages are opened. The valve 54X can be transitioned among a parallel state in which the serial flow passage 63 is closed while the other flow passages are opened, a serial state in which the suction port flow passage 54A positioned upstream (the inlet 11 side) of the valve 54X is closed while the other flow passages are opened, and a bypass state in which the suction port flow passage 54A positioned downstream of the valve 54X is closed while the other flow passages are opened.

A valve 81 is provided in the inlet direct-connecting flow passage 41 positioned downstream of the position P_{52A}. Similarly, a valve 82 is provided in the suction port flow passage 52A positioned downstream of the valve 52X. A valve 83 is provided in the suction port flow passage 53A positioned downstream of the valve 53X.

The column bypass flow passage 71 connects between the valve 81 and the suction port flow passage 52A positioned between the valve 82 and the valve 52X. Similarly, the column bypass flow passage 72 connects between the valve 82 and the suction port flow passage 53A positioned between the valve 83 and the valve 53X. The column bypass flow passage 73 connects between the valve 83 and the suction port flow passage 54A positioned between the micro pump 15 and the valve 54X.

The valve 81 can be transitioned among a normal state in which the column bypass flow passage 71 is closed while the other flow passages are opened, a bypass state in which the inlet direct-connecting flow passage 41 positioned downstream of the valve 81 is closed while the other flow passages are opened, and a closed-off state in which the inlet direct-connecting flow passage 41 positioned upstream of the valve 81 is closed while the other flow passages are opened. The valve 82 can be transitioned among a normal state in which the column bypass flow passage 72 is closed while the other flow passages are opened, a bypass state in which the suction port flow passage 52A positioned downstream of the valve 82 is closed while the other flow passages are opened, and a closed-off state in which the suction port flow passage 52A positioned upstream of the valve 82 is closed while the other flow passages are opened. The valve 83 can be transitioned among a normal state in which the column bypass flow passage 73 is closed while the other flow passages are opened, a bypass state in which the suction port flow passage 53A positioned downstream of the valve 83 is closed while the other flow passages are opened, and a closed-off state in which the suction port flow passage 53A positioned upstream of the valve 83 is closed while the other flow passages are opened.

Note that the opening and closing mechanism is configured by the valves 52X to 54X, 51Y to 53Y, and 81 to 83. Moreover, a first flow passage forming part is configured by the suction port flow passages 52A to 54A and the valves 52X to 54X. A second flow passage forming part is configured by the serial flow passages 61 to 63 and the valves 51Y to 53Y. A third flow passage forming part is configured by the discharge port flow passages 51B to 53B and the valves 51Y to 53Y. Furthermore, a row bypass flow passage is configured by the suction port flow passages 52A to 54A.

As shown in Fig. 9, a sensor unit 45 is provided in the vicinity of the outlet 12 in the outlet direct-connecting flow passage 42. The sensor unit 45 includes: a pressure sensor 45P for detecting the static pressure P of a fluid in the vicinity of the outlet 12 in the outlet direct-connecting flow passage 42; and a flow rate sensor 45Q for detecting the flow rate Q of a fluid in the vicinity of the outlet 12 in the outlet direct-connecting flow passage 42.

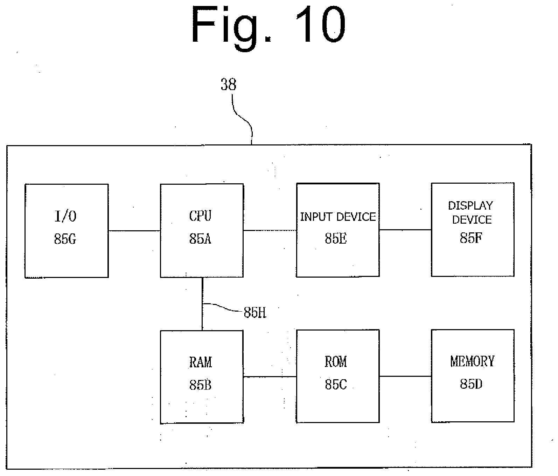

The controller 38 includes, as a hardware configuration, a CPU 85A, a first memory medium 85B, a second memory medium 85C, a third memory medium 85D, an input device 85E, a display device 85F, an input and output interface 85G, and the bus 85H (see Fig. 10). The CPU 85A is what is called a central processing unit and executes various programs to obtain various functions of the controller 38. The first memory medium 85B is what is called a RAM (Random Access Memory) and is a memory used as a work area for the CPU 85A. The second memory medium 85C is what is called a ROM (Read Only Memory) and is a memory for storing a basic operating system (OS) executed by the CPU 85A. The third memory medium 85D is configured by a hard disk device incorporating a magnetic disk, a disk device accommodating a CD, a DVD, or a BD, a nonvolatile semiconductor flash memory device, and the like. The third memory medium 85D saves various programs to be executed by the CPU 85A, sensing data from the sensors, etc. The input device 85E is an input key, a keyboard, a mouse, or the like and is a device used for inputting a variety of information. The display device 85F is a display and displays various operating states. The input and output interface 85G supplies predetermined power to the valves 52X to 54X, 51Y to 53Y, and 81 to 83, the switching valve 41Z, the respective micro pumps 15 (see Fig. 8), and the respective sensor units 36 and 45 (see Figs. 5 and 9). The input and output interface 85G also inputs and outputs predetermined control signals to and from the valves 52X to 54X, 51Y to 53Y, and 81 to 83, the switching valve 41Z, the respective sensor units 36 and 45, and the respective micro pumps 15. Furthermore, the input and output interface 85G can also obtain data such as a program from an external personal computer or output measurement results to such a personal computer. The bus 85H is a line used for integrally connecting the CPU 85A, the first memory medium 85B, the second memory medium 85C, the third memory medium 85D, the input device 85E, the display device 85F, the input and output interface 85G, and the like to achieve communication thereamong.

It is preferable that the line 85H be formed so as to be exposed to the depressed portion 13K (see Fig. 3) provided on the inlet-side housing plate 13A (see Fig. 9). As a result of this, when the micro pump 15 is housed in the depressed portion 13K, the external power-supply terminal 37 of the micro pump 15 housed in the depressed portion 13K is electrically connected to the line 85H. In this manner, the housing of the micro pump 15 in the depressed portion 13K achieves the wiring to the micro pump 15. Note that it is only necessary that the line 85H is formed so as to be exposed to the depressed portion on at least one of the inlet-side housing plate 13A and the outlet-side housing plate 13B. In the depressed portion on the other one of the inlet-side housing plate 13A and the outlet-side housing plate 13B, a biasing member (a plate spring, a coil spring, or the like) 85J for biasing the external power-supply terminal 37 of the micro pumps 15 housed in the depressed portion toward the one of the depressed portions may be provided. If the biasing member is conductive, the biasing member and the line 85H may be electrically connected to each other.

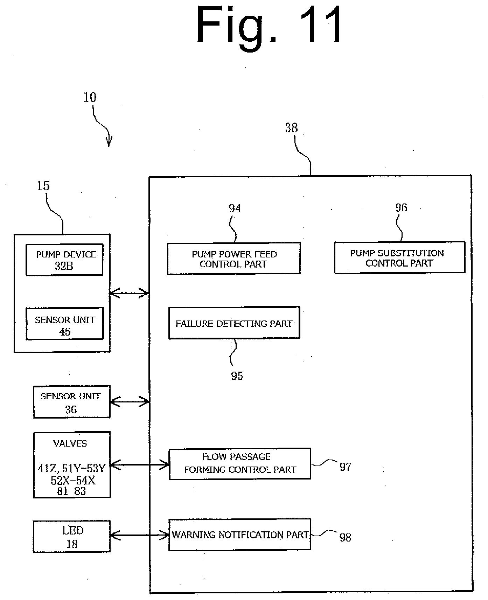

When a control program stored in the controller 38 is executed by the CPU 85A, the controller 38 functions as a pump power feed control part 94, a failure detecting part 95, a pump substitution control part 96, a flow passage forming control part 97, and a warning notification part 98 as shown in Fig. 11.

The pump power feed control part 94 feeds power to the pump device 32 of a predetermined micro pump 15 according to operating conditions set in advance by an operation of the input device 85E or the like. The operating conditions refer to conditions under which a fluid with a desired static pressure P and a desired flow rate Q is outputted from the outlet 12 (see Fig. 5) of the pump unit 10, for example.

The failure detecting part 95 reads sensing signals from the respective sensors of the sensor unit 36 provided in the micro pump 15 and determines whether or not a measured value indicated by the sensing signal exceeds an acceptable range. Herein, the acceptable range refers to values between the upper limit value and the lower limit value set by an operation of the input device 85E or the like. The upper limit value and the lower limit value are set so that the static pressure P and the flow rate Q of a fluid exiting from the micro pump 15 failing to exert the expected capability due to the deterioration or failure of the pump device 32 each fall outside the acceptable range. Moreover, if all the measured values from the respective sensors fall within the acceptable range, the failure detecting part 95 determines that the micro pump 15 in which those measured values are obtained is in a normal state. If at least one of the measured values from the respective sensors exceeds the acceptable range, the failure detecting part 95 determines that the micro pump 15 in which such a measured value is obtained is in a failure state. Furthermore, the failure detecting part 95 outputs a failure signal. The failure signal contains information about an identifier of the micro pump 15 determined as failure (for example, the micro pump arranged in the i-th row × the j-th row) .

The pump substitution control part 96 determines whether or not the failure signal is outputted from the failure detecting part 95. Also, the pump substitution control part 96 can receive the failure signal. Moreover, the pump substitution control part 96 can load power feed list information about the micro pumps 15 fed by the pump power feed control part 94 from the pump power feed control part 94. Furthermore, the pump substitution control part 96 determines if the micro pump 15 in a standby state is present or not. Herein, the standby state refers to a state in which determination as failure has not been made (normal state) and power supply is being stopped (power-feeding stopped state).

With reference to the sensing signals from the sensor units 36 and 45, the flow passage forming control part 97 performs opening and closing operations of the opening and closing mechanism, i.e., the valves 52X to 54X, 51Y to 53Y, and 81 to 83, so that the flow rate Q and the static pressure P at the outlet 12 are equal to or close to predetermined values.

The warning notification part 98 controls the turning ON and OFF of the light-emitting diode 18. Note that a buzzer or the like may be used as a warning device without being limited to the light-emitting diode 18.

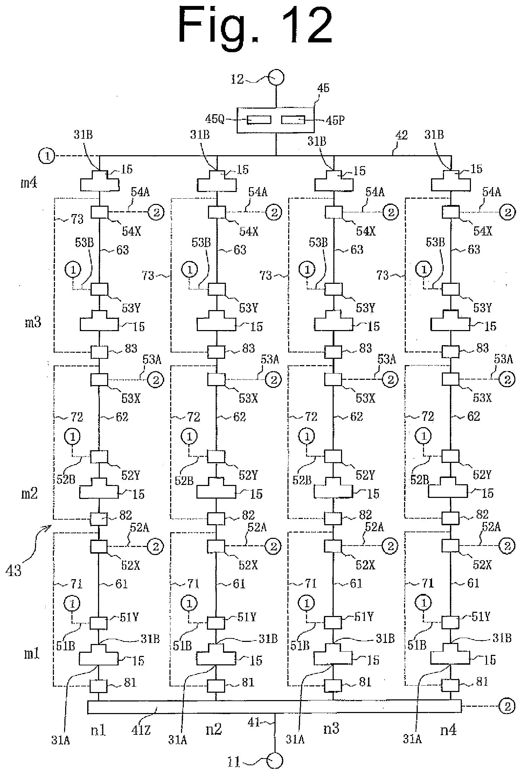

Next, control examples of the pump unit 10 performed by the controller 38 will be described. The pump power feed control part 94 turns all the micro pumps 15 to an operating state. If the flow passage forming control part 97 sets the valves 81 to 83 to the normal state and sets the valves 52X to 54X and 51Y to 53Y to the serial state, a fluid entering through the inlet 11 then goes through the micro pumps 15 arranged in the column direction and exits from the outlet 12 (see Fig. 12). As the number of the micro pumps 15 the fluid passed through is increased in this manner, the static pressure P of the fluid exiting from the outlet 12 is increased in preference to the flow rate Q. Thus, the pump unit 10 is in a state in which the static pressure P of the fluid exiting from the outlet 12 is increased in preference to the flow rate Q (pressure preferential transporting state).

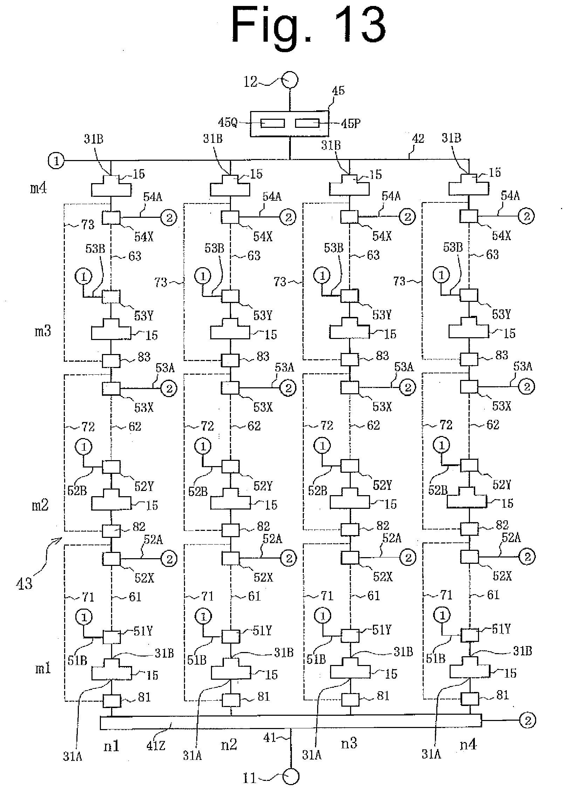

If the flow passage forming control part 97 sets the switching valve 41Z to the parallel state, the valves 81 to 83 to the normal state, and the valves 52X to 54X and 51Y to 53Y to the parallel state, a fluid entering through the inlet 11 then branches at each of the suction ports of the micro pumps 15 and enters into the micro pumps 15. The fluids exited from the discharge ports of the micro pumps 15 join together again and exit from the outlet 12 (see Fig. 13). As a result of this, the pump unit 10 is in a state in which the flow rate Q of the fluid exiting from the outlet 12 is increased in preference to the static pressure P (flow rate preferential transporting state) .

If the flow passage forming control part 97 sets the switching valve 41Z to the parallel state, the valves 81 to 83 to the normal state, the valves 52X to 54X and 51Y to 52Y to the serial state, and the valve 53Y to the parallel state, the flow rate Q and the static pressure P of the fluid exiting from the outlet 12 each take a value between the aforementioned two examples.

Controlling the valves 52X to 54X and 51Y to 52Y separately in this manner allows the fluid exiting from the outlet 12 to have a desired flow rate Q and a desired static pressure P.

Here, if the micro pumps 15 fed by the pump power feed control part 94 include the micro pump 15 in a state in which the pump device 32 is not operating normally (hereinafter referred to as a failure state), the flow rate Q and the static pressure P of the fluid exiting from the outlet 12 cannot be controlled with high accuracy.

Therefore, it is preferable that a spare micro pump 15 substitutable for the micro pump 15 in the failure state be provided in the pump unit 10 in advance.

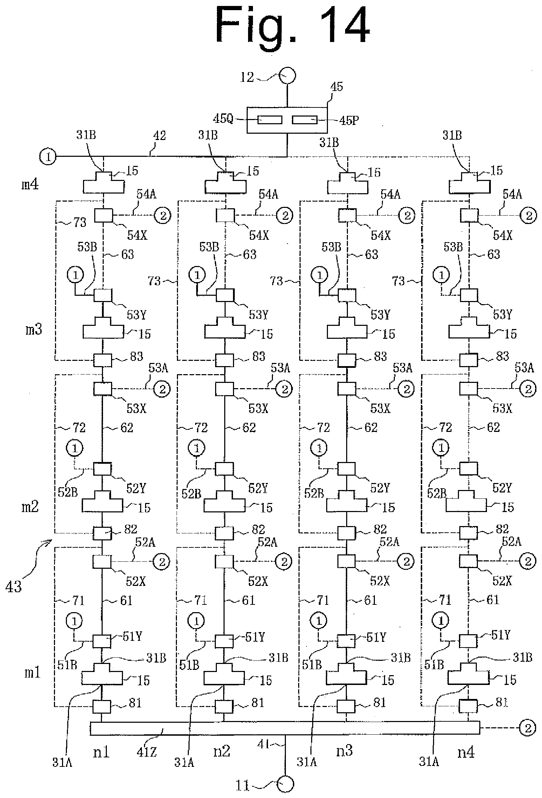

For example, as shown in Fig. 14, if the micro pumps 15 are arranged in a lattice pattern (4 rows × 4 columns), all the micro pumps 15 positioned in the fourth column and the fourth row are used as the spare micro pumps 15.

First, the pump power feed control part 94 feeds power only to the micro pumps 15 in the first to third rows × the first to third columns. The micro pumps 15 in the first to third rows × the first to third columns are therefore in the operating state while the spare micro pumps 15 are in the power-feeding stopped state. The flow passage forming control part 97 sets the switching valve 41Z to the parallel state, the valves 81 to 83 in the first to third columns to the normal state, the valves 81 to 83 in the fourth column to the closed-off state, the valves 51Y to 52Y in the first to third columns to the serial state, the valves 53Y in the first to third columns to the parallel state, and the valves 52X to 54X in the first to third columns to the serial state. In addition, the valves 54X in the first to third columns and the valves 52X to 54X and the valves 51Y to 53Y in the fourth column may be set to the serial state. As a result of this, the pump unit 10 is in the state in which the static pressure P of the fluid exiting from the outlet 12 is increased in preference to the flow rate Q.

Here, the controller 38 performs the following control. The failure detecting part 95 reads the sensing signals from the respective sensor units 36. The timing at which the sensing signals are read may occur periodically or continuously. The failure detecting part 95 determines whether or not the measured values indicated by the read sensing signals fall outside the acceptable range. If the measured values each fall within the acceptable range, the failure detecting part 95 determines that the micro pump 15 from which the sensing signals are read is in the normal state. If the measured values each fall outside the acceptable range, on the other hand, the failure detecting part 95 determines that the micro pump 15 from which the sensing signals are read is in the failure state. If it is determined that there is the micro pump 15 in the failure state, the failure detecting part 95 then outputs the failure signal.

The pump substitution control part 96 determines whether or not the failure signal is outputted from the failure detecting part 95. If the pump substitution control part 96 determines that "the failure signal has been outputted from the failure detecting part 95," the pump substitution control part 96 then determines "whether or not there is the micro pump 15 in the standby state among the micro pumps 15 contained in the pump unit 10." If the pump substitution control part 96 determines that there is the micro pump 15 in the standby state, the pump power feed control part 94 then starts feeding power to the micro pump 15 selected from the micro pumps 15 in the standby state (hereinafter referred to as a selected micro pump 15). Note that the pump power feed control part 94 preferably stops feeding power to the micro pump 15 determined as being in the failure state. Next, the flow passage forming control part 97 performs the opening and closing operations of the valves 51Y to 53Y, 52X to 54X, and 81 to 83 so that the fluid flows through the selected micro pump 15 instead of the micro pump 15 determined as failure. This allows the fluid with a desired static pressure P and a desired flow rate Q to be outputted from the outlet 12 of the pump unit 10 even when the micro pump 15 in the failure state is present in the pump unit 10.

Control contents performed by the flow passage forming control part 97 for allowing the spare micro pump 15 to be used instead of the micro pump 15 in the failure state will be described next.

First, if it is determined that the micro pump 15 in the second row × the third column is in the failure state, the flow passage forming control part 97 selects any micro pump 15 from among the spare micro pumps 15 in the standby state.

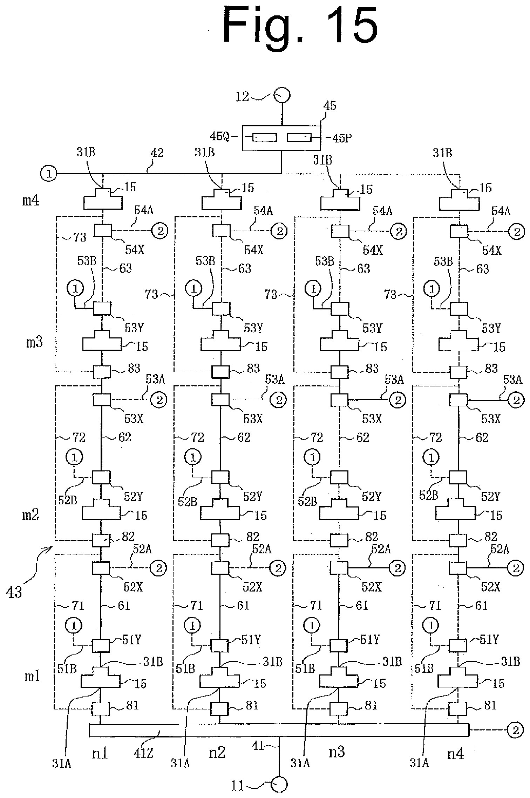

Here, if the micro pump 15 in the second row × the fourth column is selected as the substitution micro pump 15, the flow passage forming control part 97 sets the valve 52X in the third column and the valve 53X in the fourth column to the bypass state, the valve 52X in the fourth column and the valve 53X in the third column to the parallel state, the valve 52Y in the fourth column to the serial state, and the valve 82 in the fourth column to the normal state. As a result of this, the fluid having passed through the micro pump 15 in the first row × the third column passes through the micro pump 15 in the second row × the fourth column instead of the micro pump 15 in the second row × the third column. Thereafter, the fluid passes through the micro pump 15 in the third row × the third column (see Fig. 15). Thus, the fluid with the expected flow rate Q and the expected static pressure P can be outputted from the outlet 12.

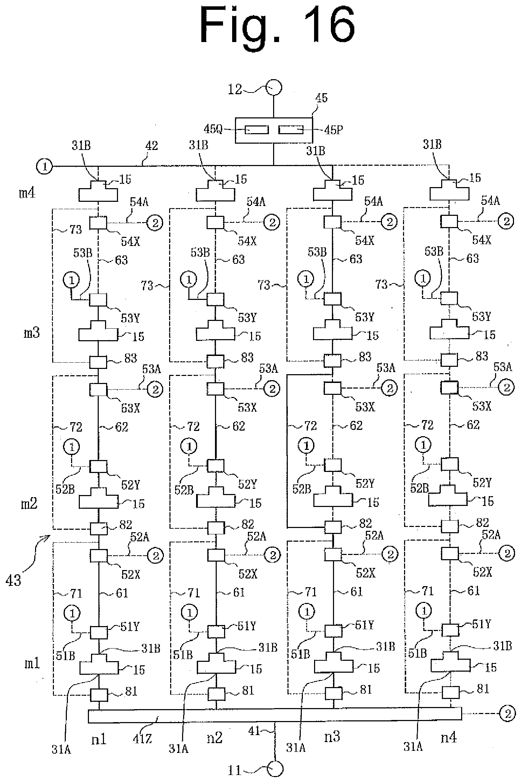

If the micro pump 15 in the fourth row × the third column is selected as the substitution micro pump 15, the flow passage forming control part 97 sets the valve 82 in the third column to the bypass state, the valve 83 in the third column to the normal state, and the valves 53Y and 54X in the third column to the serial state. Note that it is preferable that the valve 53X in the third column be in the serial state. As a result of this, the fluid having passed through the micro pump 15 in the first row × the third column passes through the micro pump 15 in the third row × the third column without passing through the micro pump 15 in the second row × the third column. Thereafter, the fluid passes through the micro pump 15 in the fourth row × the third column (see Fig. 16). Thus, the fluid with the expected flow rate Q and the expected static pressure P can be outputted from the outlet 12.

If the pump substitution control part 96 determines that there is no micro pump 15 in the stopped state, on the other hand, the warning notification part 98 can give a notification of an abnormal state in the pump unit 10 by controlling the turning ON and OFF of the light-emitting diode 18. As a result of this, the use of the pump unit 10 which cannot output the fluid with the desired static pressure P and the desired flow rate Q can be avoided.

As described above, according to the pump unit 10, the micro pumps 15 are arranged in a lattice pattern and by means of the flow passage forming mechanism, i.e., the middle flow passage 43 and the opening and closing mechanism (the valves) provided in the middle flow passage 43, rational combinations about the serial connection and parallel connection of the micro pumps 15 can be controlled. Consequently, even for an application in which a single micro pump 15 fails to achieve a sufficient flow rate and a sufficient static pressure, a plurality of micro pumps 15 can be used in combination. Therefore, such micro pumps 15 can be used in a similar manner to the conventional blowers or syringe pumps. Moreover, due to the small size of the micro pump 15, even when a plurality of such micro pumps 15 are arranged, they can be configured to be smaller and lighter than the conventional blowers or the like. In particular, various variations about a combination of the number of parallel connections and the number of serial connections can be digitally controlled by the turning ON and OFF of the micro pumps 15 or the control of the opening and closing mechanism (valves). Thus, the control configuration thereof can be extremely simplified. Furthermore, in the case of the conventional blowers or syringe pumps, if one of them is broken down, the entire fluid transportation is disrupted. According to the above-described pump unit 10, however, even if an individual micro pump 15 is broken down, the other micro pumps 15 can make up for the broken micro pump 15. Thus, reliability or safety can be also enhanced.

Particularly in the pump unit 10, the number of the micro pumps 15 that belong to the upstream row is equal to or smaller than the number of the micro pumps 15 that belong to the downstream row in the pressure preferential transporting state in which the micro pumps 15 are connected in series. Consequently, the unnecessary operation of the micro pumps 15 can be suppressed, thereby making it possible to reduce power consumption. This is especially suitable for a battery-driven application, for example.

Furthermore, the pump unit 10 collectively switches the connection relationship of the entire micro pumps 15 arranged at each row. Consequently, the configuration of the valves is simplified, thereby improving the maintainability thereof.

Note that a single or a plurality of inlets 11 may be provided in the pump unit 10. The plurality of inlets 11 may be connected to the inlet direct-connecting flow passage 41 or directly connected to the micro pumps 15 that belong to the most upstream row group 21. Moreover, a single or three or more middle row groups may be provided.

In the above-described embodiment, the most upstream row group 21, the middle row groups 22 and 23, and the most downstream row group 24 are arranged in this order in the housing 13. However, the present invention is not limited thereto. For example, the order of the most upstream row group 21, the most downstream row group 24, and the middle row groups 22 and 23, the order of the most downstream row group 24, the middle row groups 22 and 23, and the most upstream row group 21, or the like is possible.

While the micro pumps 15 are arranged in a lattice pattern in the housing 13 in the above-described embodiment, the present invention is not limited thereto. The micro pumps 15 may be arranged to form a single row or a single column.

Moreover, while the micro pumps 15 are fitted into the housing 13 in the above-described embodiment, the present invention is not limited thereto. The micro pumps 15 and the housing 13 may be integrally formed.

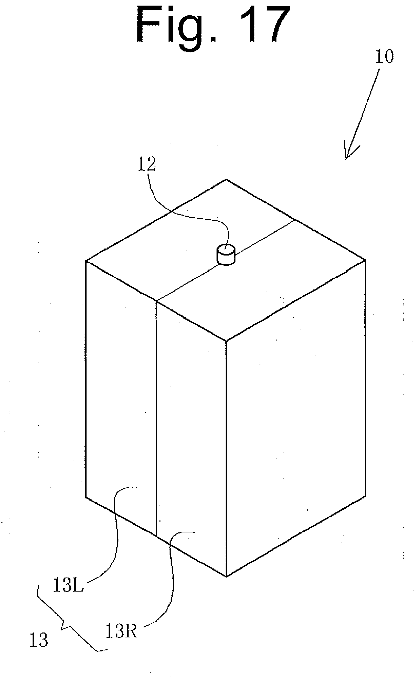

While the micro pumps 15 are arranged on a plane in a lattice pattern in the above-described embodiment, the present invention is not limited thereto. A plurality of micro pumps 15 may be arranged so as to overlap one another. For example, the micro pumps 15 may be stacked in such a manner that the inlet 11 of the second micro pump 15 is positioned above the outlet 12 of the first micro pump 15 (see Figs. 17 to 20).

The pump unit 10 shown in Figs. 17 to 18 includes: the housing 13 with the inlet 11 and the outlet 12; and a pump unit 15 housed in the housing 13. The housing 13 having a pump unit housing hole 13X for housing the pump unit 15 is configured by a first housing forming block 13L and a second housing forming block 13R. A predetermined depressed part is formed in each of the first housing forming block 13L and the second housing forming block 13R. The first housing forming block 13L and the second housing forming block 13R are fitted together in such a manner that the depressed parts face each other to form the pump unit housing hole 13X.

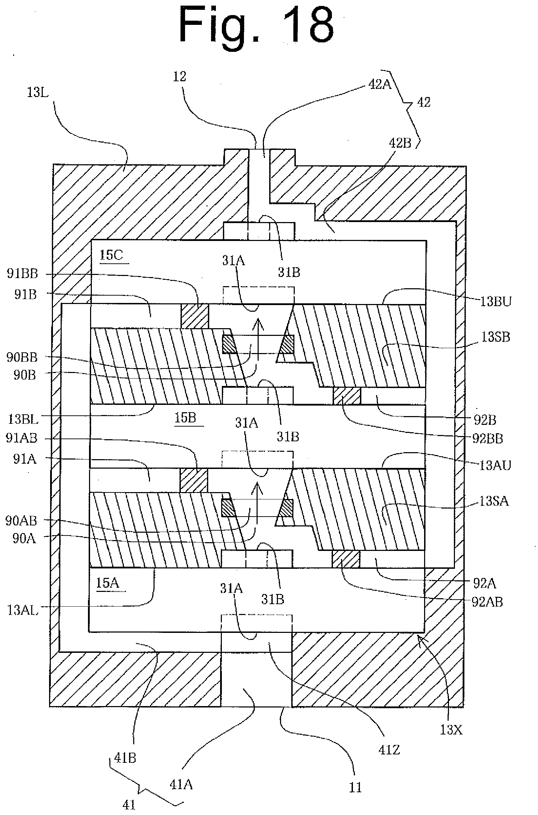

As shown in Figs. 18 to 19, micro pumps 15A, 15B, and 15C arranged in this order from the inlet 11 toward the outlet 12 in the housing 13, a flow passage block 13SA disposed between the micro pump 15A and the micro pump 15B, and a flow passage block 13SB disposed between the micro pump 15B and the micro pump 15C are arranged in the housing 13.

Moreover, in the housing 13, the inlet direct-connecting flow passage 41 connecting between the suction port 31A of the micro pump 15A and the inlet 11 and the outlet direct-connecting flow passage 42 connecting between the discharge port 31B of the micro pump 15C and the outlet 12 are formed. The inlet direct-connecting flow passage 41 includes: a direct-connecting passage 41A directly connecting the suction port 31A of the micro pump 15A with the inlet 11; and a branched passage 41B branched from the direct-connecting passage 41A. The branched passage 41B extends to the vicinity of the suction port 31A of the micro pump 15C along the micro pumps 15A, 15B, and 15C. The outlet direct-connecting flow passage 42 includes: a direct-connecting passage 42A directly connecting the discharge port 31B of the micro pump 15C with the outlet 12; and a branched passage 42B branched from the direct-connecting passage 42A. The branched passage 42B extends to the vicinity of the discharge port 31B of the micro pump 15A along the micro pumps 15C, 15B, and 15A.

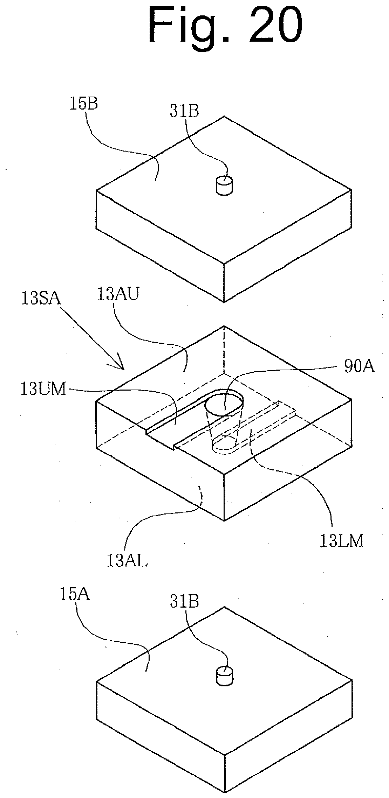

As shown in Figs. 18 and 20, the flow passage block 13SA is formed in the shape of a rectangular parallelepiped. In the flow passage block 13SA, a serial flow passage 90A directly connecting the discharge port 31B of the micro pump 15A with the suction port 31A of the micro pump 15B; a serial valve 90AB for opening and closing the serial flow passage 90A; a discharge-side parallel flow passage 92A directly connecting the serial flow passage 90A closer to the discharge port 31B than the serial valve 90AB with the branched passage 42B; a discharge-side parallel valve 92AB for opening and closing the discharge-side parallel flow passage 92A; a suction-side parallel flow passage 91A directly connecting the serial flow passage 90A closer to the suction port 31A than the serial valve 90AB with the branched passage 41B; and a suction-side parallel valve 91AB for opening and closing the suction-side parallel flow passage 91A are formed. Note that the diagrammatic illustration of the valves 90AB, 91AB, and 92AB is omitted in Fig. 20 in order to avoid being complicated. The flow passage block 13SB is similar to the flow passage block 13SA. More specifically, the flow passage block 13SB is formed in the shape of a rectangular parallelepiped, and a serial flow passage 90B directly connecting the discharge port 31B of the micro pump 15B with the suction port 31A of the micro pump 15C; a serial valve 90BB for opening and closing the serial flow passage 90B; a discharge-side parallel flow passage 92B directly connecting the serial flow passage 90B closer to the discharge port 31B than the serial valve 90BB with the branched passage 42B; a discharge-side parallel valve 92BB for opening and closing the discharge-side parallel flow passage 92B; a suction-side parallel flow passage 91B directly connecting the serial flow passage 90B closer to the suction port 31A than the serial valve 90BB with the branched passage 41B; and a suction-side parallel valve 91BB for opening and closing the suction-side parallel flow passage 91B are formed.

The serial flow passage 90A is formed so as to run through from a discharge port side surface 13AL of the flow passage block 13SA facing the discharge port 31B of the micro pump 15A to a suction port side surface 13AU of the flow passage block 13SA facing the suction port 31A of the micro pump 15B. Since the housing of the micro pump 15A is in contact with the discharge port side surface 13AL in the housing 13, a groove 13LM formed on the discharge port side surface 13AL and the micro pump 15A together form the discharge-side parallel flow passage 92A. Since the housing of the micro pump 15B is in contact with the suction port side surface 13AU in the housing 13, a groove 13UM formed on the suction port side surface 13AU and the micro pump 15B together form the suction-side parallel flow passage 91A. Similarly, the serial flow passage 90B is formed so as to run through from a discharge port side surface 13BL of the flow passage block 13SB facing the discharge port 31B of the micro pump 15B to a suction port side surface 13BU of the flow passage block 13SB facing the suction port 31A of the micro pump 15C. Since the housing of the micro pump 15B is in contact with the discharge port side surface 13BL in the housing 13, a groove formed on the discharge port side surface 13BL and the micro pump 15B together form the discharge-side parallel flow passage 92B. Since the housing of the micro pump 15C is in contact with the suction port side surface 13BU in the housing 13, a groove formed on the suction port side surface 13BU and the micro pump 15C together form the suction-side parallel flow passage 91B.

The opening and closing operations of the switching valve 41Z, the serial valves 90AB and 90BB, the suction-side parallel valves 91AB and 91BB, and the discharge-side parallel valves 92AB and 92BB are performed by the controller 38 (see Fig. 7).

Functions of the pump unit 10 shown in Figs. 17 to 20 will be described next.

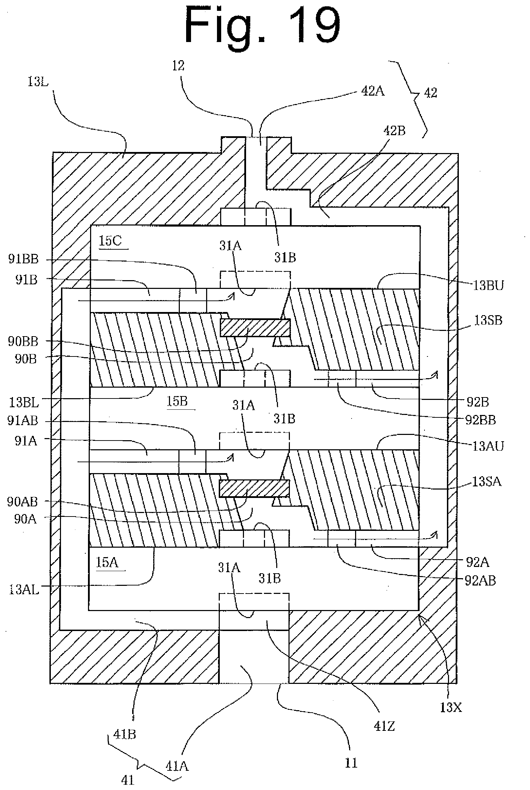

The switching valve 41Z is set to the parallel state, the serial valves 90AB and 90BB are set to a closed state, and the suction-side parallel valves 91AB and 91BB and the discharge-side parallel valves 92AB and 92BB are set to an open state (see Fig. 19). A fluid entering through the inlet 11 is distributed through the inlet direct-connecting flow passage 41, the suction-side parallel flow passage 91A, and the suction-side parallel flow passage 91B. The distributed fluids are sucked into the suction ports 31A of the micro pumps 15A to 15C, respectively. In each of the micro pumps 15A to 15C, the pump device 32 (see Fig. 5) compresses the fluid sucked in from the suction port 31A. The fluids compressed in the micro pumps 15A to 15C exit from the discharge ports 31B, join together through the discharge-side parallel flow passage 92A, the discharge-side parallel flow passage 92B, and the outlet direct-connecting flow passage 42, and then exit from the outlet 12.

The switching valve 41Z is set to the serial state, the serial valves 90AB and 90BB are set to the open state, and the suction-side parallel valves 91AB and 91BB and the discharge-side parallel valves 92AB and 92BB are set to the closed state (see Fig. 18). A fluid entering through the inlet 11 is sucked into the suction port 31A of the micro pump 15A through the inlet direct-connecting flow passage 41. In the micro pump 15A, the pump device 32 (see Fig. 5) compresses the fluid sucked in from the suction port 31A. The fluid compressed in the micro pump 15A exits from the discharge port 31B and is sucked into the suction port 31A of the micro pump 15B through the serial flow passage 90A. The fluid sucked in from the suction port 31A of the micro pump 15B is compressed by the pump device 32 (see Fig. 5) and then sucked into the suction port 31A of the micro pump 15C through the discharge port 31B and the serial flow passage 90B. Similarly, the fluid sucked in from the suction port 31A of the micro pump 15C is compressed by the pump device 32 (see Fig. 5) and then exits from the outlet 12 through the discharge port 31B and the outlet direct-connecting flow passage 42.

According to the pump unit 10, the static pressure P and the flow rate Q of the fluid exiting from the outlet 12 can be appropriately controlled by means of the opening and closing operations of the switching valve 41Z, the serial valves 90AB and 90BB, the suction-side parallel valves 91AB and 91BB, and the discharge-side parallel valves 92AB and 92BB.

Moreover, since the groove 13LM formed on the discharge port side surface 13AL and the micro pump 15A together form the discharge-side parallel flow passage 92A, time and effort required to form the discharge-side parallel flow passage 92A can be saved. Similarly, since the groove 13UM formed on the suction port side surface 13AU and the micro pump 15B together form the suction-side parallel flow passage 91A, time and effort required to form the suction-side parallel flow passage 91A can be saved.

This applies also to the housing 13 shown in Fig. 9. It is preferable that the inlet-side housing plate 13A be formed by flow passage forming plates 13AA to 13AD. Each of the flow passage forming plates 13AA to 13AD has a through hole formed in a thickness direction thereof at a predetermined position. Moreover, each of the flow passage forming plates 13AA to 13AD has a groove at a predetermined position on a surface facing another flow passage forming plate. When the flow passage forming plates 13AA to 13AD are fitted together in a stacked manner, the through holes and the grooves formed in the flow passage forming plates 13AA to 13AD form the respective flow passages 41, 52A to 54A, and 71 to 73 and upstream portions of the respective flow passages 61 to 63. Similarly, it is preferable that the outlet-side housing plate 13B be formed by flow passage forming plates 13BA to 13BB. Each of the flow passage forming plates 13BA to 13BB has a through hole formed in a thickness direction thereof and a groove formed on a surface facing another flow passage forming plate at predetermined positions. When the flow passage forming plates 13BA to 13BB are fitted together in a stacked manner, the through holes and the grooves formed in the flow passage forming plates 13BA to 13BB form the respective flow passages 42 and 51B to 53B and downstream portions of the respective flow passages 61 to 63.

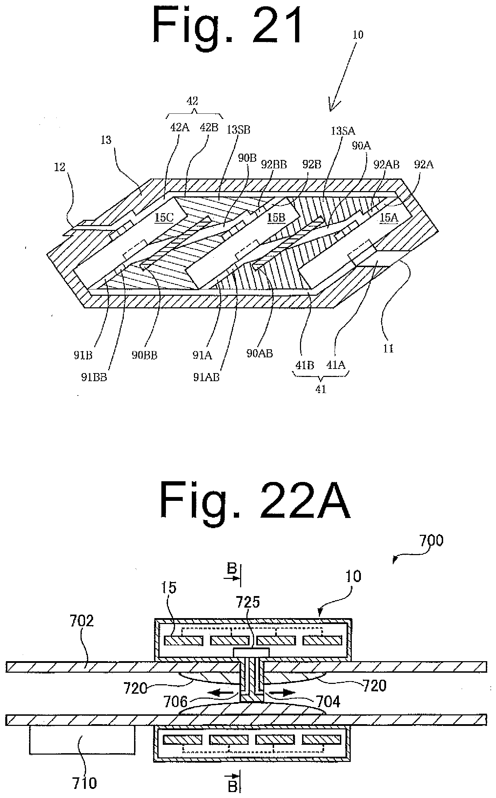

While the outlet 12 of the first micro pump 15 and the inlet 11 of the second micro pump 15 are arranged so as to directly face each other in the above-described embodiment, the present invention is not limited thereto. For example, as shown in Fig. 21, a plurality of micro pumps 15 may be stacked one another in an oblique direction. The pump unit 10 configured by the plurality of micro pumps 15 stacked one another in an oblique direction can be placed in a small space such as an interspace between objects.

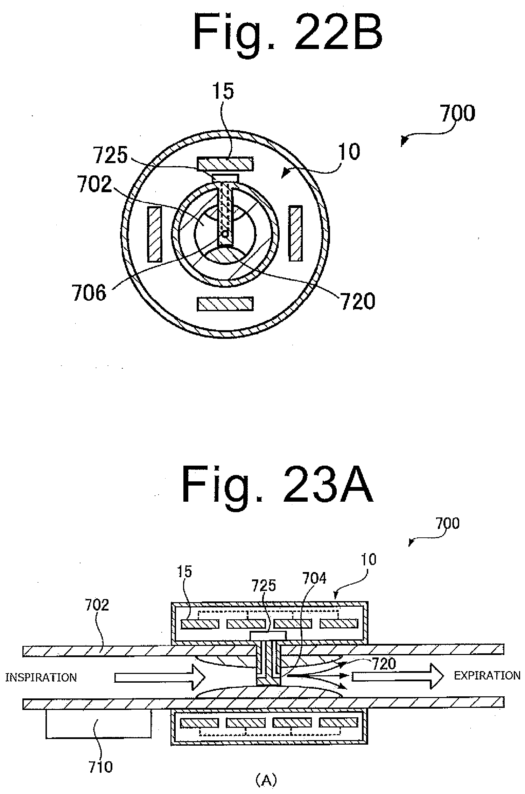

An example in which the pump unit 10 is applied to a respiratory assistance device 700 for medical use is shown in Figs. 22A and 22B. The respiratory assistance device 700 is configured by including: a flow passage 702 through which air for respiration passes; an expiratory nozzle 704 and an inspiratory nozzle 706 disposed in the flow passage 702 and capable of emitting an acceleration air in an expiratory direction and in an inspiratory direction, respectively; the pump unit 10 disposed on an outer surface of the flow passage 702 in a circumferential direction thereof; and a battery 710 for driving the pump unit 10. Venturi walls 720 are disposed in the vicinity of the expiratory and inspiratory nozzles 704 and 706 disposed in the flow passage 702. Note that the battery 710 may be disposed at a remote location or may be omitted by connecting a power supply line.

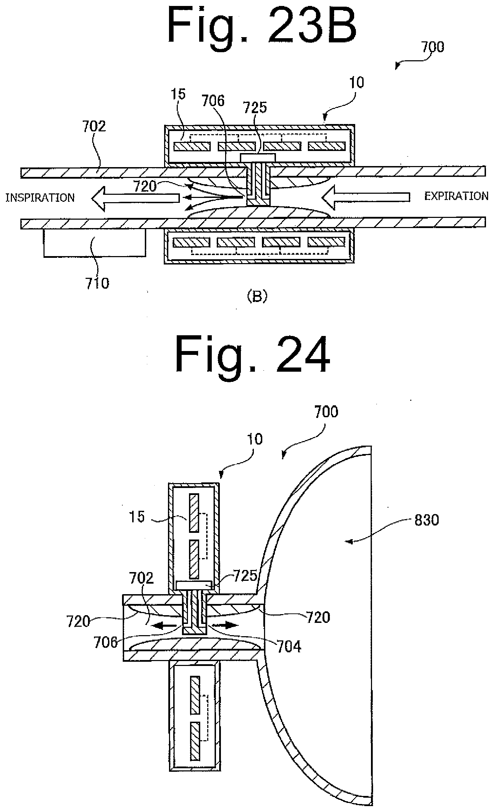

Furthermore, an expiration and inspiration switching valve 725 is disposed at the outlet 12 (see Fig. 1, hereinafter referred to as an integrated discharge port) of the pump unit 10. The expiration and inspiration switching valve 725 switches between a case where air to be discharged from the integrated discharge port is emitted from the expiratory nozzle 704 and a case where such air is emitted from the inspiratory nozzle 706. When air is emitted from the expiratory nozzle 704 as shown in Fig. 23A, such air is spread out by the Venturi wall 720, thereby setting the expiratory side to a negative pressure state. Thus, carbon dioxide discharged from the inspiratory side (lung side) is drawn into the air and such air is caused to flow in the expiratory direction. Consequently, an expiratory action can be assisted. When air is emitted from the inspiratory nozzle 706 as shown in Fig. 23B, on the other hand, such air is spread out by the Venturi wall 720, thereby setting the inspiratory side to the negative pressure state. Thus, oxygen supplied from the inspiratory side is absorbed in the air and such air is caused to flow in the inspiratory direction (lung side). Consequently, an inspiratory action can be assisted.

According to the respiratory assistance device 700, the downsized pump unit 10 is directly fixed to a pipe itself that forms the flow passage 702. Thus, the respiratory assistance device 700 can be configured in an extremely compact manner. Furthermore, due to the integral formation of the flow passage 702 and the pump unit 10, even when the flow passage 702 is moved along with a user's body movement, the flow passage 702 and the pump unit 10 move together. Thus, the connection between the expiratory and inspiratory nozzles 704 and 706 and the pump unit 10 is prevented from being cut off. Therefore, stability in the breathing assisting operation is enhanced and a user can also move his or her body more freely.

Furthermore, due to a reduced distance between the pump unit 10 and the expiratory and inspiratory nozzles 704 and 706, responsiveness of the breathing assisting operation can be enhanced.

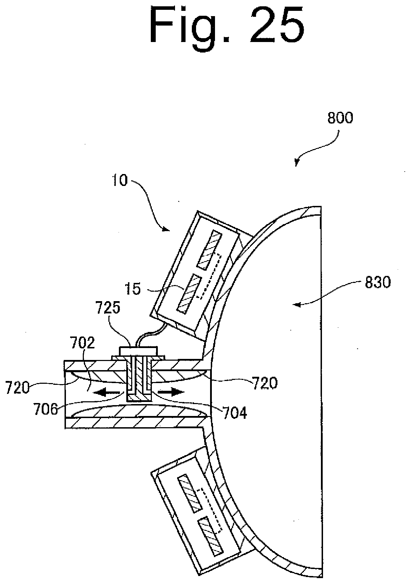

The respiratory assistance device 700 can be used continuously with an intubation tube inserted toward a windpipe through a mouth of a user. However, the respiratory assistance device 700 can alternatively be used with the flow passage 702 being connected to a nose mask 830 as shown in Fig. 24, for example. Furthermore, when applied to a nose mask, it is preferable that the pump unit 10 be directly fixed to an outer peripheral surface of the nose mask 830 as in a respiratory assistance device 800 shown in Fig. 25, for example. Such an arrangement enhances the overall stability. While the case where air is supplied to the expiratory nozzle or the inspiratory nozzle by switching a single pump unit 10 by means of the expiration and inspiration switching valve 725 has been illustrated here, two pump units 10 may be provided and connected to the expiratory nozzle and the inspiratory nozzle, respectively.

It is apparent that the pump unit and the respiratory assistance device according to the present invention are not limited to the above-described embodiments and various modifications can be made thereto without departing from the scope of the present invention.

### Industrial Applicability

The pump unit according to the present invention can be used in various applications other than the respiratory assistance device. Moreover, the respiratory assistance device according to the present invention can be utilized in order to assist the breathing of various creatures.

## Claims

1. A pump unit comprising:
a body (10) provided with an inlet (11) and an outlet (12) for a fluid; and
a pump group composed of a plurality of micro pumps (15) arranged in the body (10) on a plane so that discharge ports (31B) and suction ports (31A) of the micro pumps (15) are aligned in a same direction, for allowing a fluid entering through the inlet (11) to exit from the outlet (12) by discharging the fluid from the suction ports (31A) to the discharge ports (31B) in one direction, wherein
the body (10) includes an inlet package (13A) having the inlet (11) and an outlet package (13B) having the outlet (12) to hold the plurality of arranged micro pumps (15) between them,
the inlet package (13A) collectively holds the plurality of micro pumps (15) from a side of the suction ports (31A) and has a flow passage (41) and a switching valve (41Z) that allows the suction ports (31A) of the micro pumps (15) and the inlet (11) of the body (10) to be communicated with or closed off from each other,
the outlet package (13B) collectively holds the plurality of micro pumps (15) from a side of the discharge ports (31B) and has a structure (42) that allows the discharge ports (31B) of the micro pumps (15) and the outlet (12) of the body (10) to be communicated with or closed off from each other,
**characterized in that**
the body (10) is provided with a plurality of depressed portions (13K), each depressed portion (13K) for housing a respective micro pump (15), each micro pump (15) includes a power-feeding terminal (33) for feeding power to a pump device (32) contained therein, and
each depressed portion (13K) is provided with a line electrically connecting to the power-feeding terminal (33) of the micro pump (15) housed in the depressed portion (13K).

## Patentansprüche

1. Pumpeneinheit mit:
einem Körper (10), der mit einem Einlass (11) und einem Auslass (12) für ein Fluid bereitgestellt ist; und
einer Pumpengruppe, die aus einer Mehrzahl Mikropumpen (15) zusammengesetzt ist, die in dem Körper (10) auf einer Ebene angeordnet sind, so dass Abgabeanschlüsse (31B) und Ansauganschlüsse (31A) der Mikropumpen (15) in einer gleichen Richtung ausgerichtet sind, um es einem Fluid zu ermöglichen, durch den Einlass (11) einzutreten, um aus dem Auslass (12) durch Abgeben des Fluids von den Ansauganschlüssen (31A) zu den Abgabeanschlüssen (31B) in eine Richtung auszutreten, wobei
der Körper (10) eine Einlasspackung (13A) hat, die den Einlass (11) aufweist, und eine Auslasspackung (13B), die den Auslass (12) aufweist, um die Mehrzahl der angeordneten Mikropumpen (15) zwischen diesen zu halten,
die Einlasspackung (13A) kollektiv die Mehrzahl Mikropumpen (15) von einer Seite der Anzahl der Anschlüsse (31A) hält und einen Strömungsdurchgang (41) und ein Schaltventil (41Z) aufweist, das es ermöglicht, die Ansauganschlüsse (31A) der Mikropumpen (15) und dem Einlass (11) des Körpers (10) miteinander zu verbinden oder voneinander abzusperren,
die Auslasspackung (13B) kollektiv die Mehrzahl Mikropumpen (15) von einer Seite der Abgabeanschlüsse (31B) hält und eine Struktur (42) aufweist, die es ermöglicht, dass sie Abgabeanschlüsse (31B) der Mikropumpen (15) und der Auslass (12) des Körpers (10) miteinander in Verbindung sind oder voneinander abgesperrt sind, 7
**dadurch gekennzeichnet, dass**
der Körper (10) mit einer Mehrzahl niedergedrückter Abschnitte (13K) bereitgestellt ist, wobei jeder niedergedrückte Abschnitt (13K) zum Aufnehmen einer entsprechenden Mikropumpe (15) dient,
jede Mikropumpe (15) einen Leistungszufuhranschluss (33) zum Zuführen von Leistung zu einem darin vorhandenen Pumpengerät (32) hat, und
jeder niedergedrückte Abschnitt (13K) mit einer Leitung bereitgestellt ist, die den Leistungszufuhranschluss (33) der Mikropumpe (15), die in dem niedergedrückten Abschnitt (13K) aufgenommen ist, verbindet.

## Revendications

1. Unité de pompe comprenant :
un corps (10) prévu avec une entrée (11) et une sortie (12) pour un fluide ; et
un groupe de pompes composé d'une pluralité de micropompes (15) agencées dans le corps (10) sur un plan, de sorte que les orifices de décharge (31B) et les orifices d'aspiration (31A) des micropompes (15) sont alignés dans une même direction, pour permettre à un fluide d'entrer par l'entrée (11) pour ressortir par la sortie (12) en déchargeant le fluide des orifices d'aspiration (31A) aux orifices de décharge (31B) dans une direction, dans laquelle :
le corps (10) comprend une garniture d'entrée (13A) ayant l'entrée (11) et une garniture de sortie (13B) ayant la sortie (12) pour maintenir la pluralité de micropompes (15) agencées entre elles,
la garniture d'entrée (13A) maintient collectivement la pluralité de micropompes (15) depuis un côté des orifices d'aspiration (31A) et a un passage d'écoulement (41) et une valve de commutation (41Z) qui permet aux orifices d'aspiration (31A) des micropompes (15) et à l'entrée (11) du corps (10) d'être en communication entre eux ou fermés les uns par rapport aux autres,
la garniture de sortie (13B) maintient collectivement la pluralité de micropompes (15) depuis un côté des orifices de décharge (31B) et a une structure (42) qui permet aux orifices de décharge (31B) des micropompes (15) et à la sortie (12) du corps (10) d'être en communication entre eux ou fermés les uns par rapport aux autres,
**caractérisée en ce que** :
le corps (10) est prévu avec une pluralité de parties enfoncées (13K), chaque partie enfoncée (13K) étant prévue pour loger une micropompe (15) respective,
chaque micropompe (15) comprend une borne d'alimentation d'énergie (33) pour fournir l'énergie à un dispositif de pompe (32) contenu à l'intérieur de cette dernière, et
chaque partie enfoncée (13K) est prévue avec une ligne raccordant électriquement la borne d'alimentation d'énergie (33) de la micropompe (15) logée dans la partie enfoncée (13K).
